Europäisches Patentamt

European Patent Office

Office européen des brevets

(11) Publication number: **0 320 109**
**A1**

# EUROPEAN PATENT APPLICATION

(21) Application number: 88310316.0

(22) Date of filing: 02.11.88

(51) Int. Cl.4 **A61B 5/00**

(30) Priority: 05.11.87 GB 8725936

(43) Date of publication of application:
14.06.89 Bulletin 89/24

(84) Designated Contracting States:
DE FR GB IT NL

(71) Applicant: MediSense, Inc.
128 Sidney Street
Cambridge Massachusetts 02139(US)

(72) Inventor: Wickham, Mark David
425 Woburn Street Apartment No. 19
Lexington, Massachusetts 02173(US)
Inventor: Plotkin, Elliot Verne
36 Lye Valley
Headington Oxfordshire OX3 7ER(GB)
Inventor: Beck, Thomas William
8 Clifton Drive
Abingdon Oxfordshire OX14 1ET(GB)
Inventor: Wai-Lai Lock, Louisa Agnes
22 North Rise St. George's Fields
London W2 2YB(GB)

(74) Representative: Ruffles, Graham Keith et al
MARKS & CLERK 57-60 Lincoln's Inn Fields
London WC2A 3LS(GB)

(54) Improved sensing system.

(57) An amperometric invasive probe electrode (1) for detecting an analyte comprises a fill of electrode material (6) contained in a generally cylindrical closed tube (4) having an aperture (7) protected by a membrane cover (8).

FIG 1.

EP 0 320 109 A1

## IMPROVED SENSING SYSTEM

### Background of the Invention

This invention relates to electrochemical sensors for use in vivo.

It is well known to take from a living subject a sample of a body fluid, typically whole blood, and to subject this sample to external testing. One such form of testing involves subjecting the blood sample, or a preparation derived from the blood sample, to a biochemical or immunological assay utilising a protocol of stages with wet reagents.

Another and more recent category of external testing utilizes a mediated enzyme system and involves taking a blood or other sample and (with or without further preparation) placing the sample in contact with an active electrode and a counter electrode and/or a reference electrode. The active electrode is constituted so as to accept charge from or donate charge to a redox centre in a reaction catalysed specifically by an enzyme component in the electrode material. An electrochemical readout of the charge gives a direct measure of a specific component of blood.

For example, as exemplified in EP127958, a strip of insulating material can carry two small closely adjacent electrode areas, one being a reference or counter electrode of silver and silver chloride and the other being an active electrode containing a glucose dehydrogenase or glucose oxidase together with a mediator compound, especially a compound of the ferrocene type. When a spot of blood is placed over both electrodes, and the electrodes are connected to suitable processing circuitry, a read-out can be obtained which is related to the glucose concentration in the blood.

The present invention is a further development of such ideas but is directed specifically at in vivo sensors. In EP78636, the Figures 2 and 4 show sensor electrodes which are described as in vivo sensor electrodes. However, the provision on a production basis of in vivo sensors presents certain problems.

Firstly, the sensors must be of small invasive dimensions, since any invasive sensor element causes discomfort. Despite their small size, the sensors must nevertheless be accurate and give reliable reproducible measurements. The sensors must be biocompatible, and in particular their outer surface must not set up an irritant or other reaction.

One object of the invention is the provision of an in vivo sensor which is susceptible of mass production. Another object is a sensor of small invasive dimensions.

In one aspect, the invention provides an amperometric invasive probe electrode for detecting an analyte. The electrode comprises a fill of electrode material contained in a generally cylindrical closed tube having an aperture protected by a membrane cover.

The electrode material generates a current when the electrode is contacted with the analyte by introduction in to the human patient or other subject. Introduction in to the subject can be achieved directly by designing the invasive probe electrode as a needle capable of piercing the skin. In an alternative construction, the invasive probe electrode is introduced indirectly using a cannula or other delivery system.

The invasive probe electrode provided by the present invention is preferably a single-use or disposable electrode. It is of relatively small dimensions allowing comfortable in vivo use. For detection of the current and thus the analyte, the electrode is employed as part of an amperometric sensor system. The sensor system comprises:

(a) the amperometric invasive probe electrode;

(b) at least one further electrode comprising a reference electrode and optionally a counter electrode; and

(c) electrical circuitry for connection between the electrodes to amperometrically detect the analyte.

For the invasive probe electrodes of this invention, the interior of the tube at least in the vicinity of the aperture is filled with the electrode material. By way of illustration, the electrode material can comprise a mediated enzyme system for amperometric detection of an analyte, which can be a substrate of the enzyme.

Examples of such mediated enzyme systems are well known, and reference is illustratively made to Ep78636, EP12139, EP125867, EP12136 or EP125137. Thus, for instance, for the detection of glucose, the system might comprise a conductive mixture of carbon, glucose oxidase or glucose dehydrogenase, and a ferrocene compound.

It will be apparent that although illustrative reference is made to a glucose sensor, the electrode material of the invasive electrode can be based on any of the amperometric sensor systems known in the art, more especially including those described in the earlier European Patent Specifications. Thus, other enzymes can be utilised instead of the glucose oxidase so as to detect other analytes; groups of enzymes can be used so as to carry out a cascade reaction; or immunological reactions or other specific binding reactions can be used (for example, a DNA probe/target can be detected by the perturbation effect upon a known level of enzyme-catalysed reaction). Furthermore, the invention is not restricted to mediated systems, nor indeed to enzyme systems. Generally, the reader is referred to Biosensors Fundamentals and Applications, edited by Turner, Karube and Wilson, OUP 1987.

The electrode material is contained within the closed tube, made for example of stainless steel, and having an aperture, preferably a single aperture, formed for example in the side wall of the tube. The aperture is covered by the membrane cover, preferably itself formed as a tube or cast in situ. Suitable membranes are biocompatible permeable polymer restraining outward passage of the electrode material and allowing inward passage of the analyte.

Typically, the membrane is such that it is permeable to the smaller dissolved components of whole blood, or of other bodily fluids, but excludes for example living cells or large scale cellular debris and prevents outward transfer of electrode components (such as enzyme) to the tissues. If desired, the membrane can be designed to exclude large molecules.

A specific type of semi-permeable, microporous membrane, known as a phase inversion membrane, is particularly useful for the membrane.

A phase inversion membrane is suitably prepared by the precipitation of a polymer from solution in a controlled manner, for example either by evaporation of a solvent or by addition of or contact with a non-solvent bath, such as by extrusion. Such methods produce a spongy porous structure, and the membrane pore size can be controlled by changing one or more of the conditions under which the membrane is formed, the concentration and the composition of the casting solution. The pore size and general porosity is thus controlled to give the required semi-permeable properties. Typically, the phase-inversion procedure gives an asymmetric membrane, and is used with smaller pores towards the inside. Membrane materials can include polysulphone, polyethersulphone, polysulphone/polyvinylpyrrolidone, nylon, sulphonated polysulphone, cellulose acetate or polyurethane. Organic solvents can be selected to give a solution, and water is a typical non-solvent to achieve gelation.

In one preferred embodiment, the membrane is cast in situ at the aperture. In an alternative preferred embodiment, the membrane is cast separately as a tube and positioned around the aperture. Because the phase-inversion membrane has a spongy structure, it has been found that when the membrane is immersed in a suitable solvent (which can be a mixed solvent), the membrane will shrink in all dimensions. This phenomenon can be used to shrink a tubular membrane over an aperture in the side wall and hold it in place, if necessary until adhesive is applied. This procedure also serves to define more precisely the area of membrane through which metabolites can diffuse into the interior of the needle.

Solvent-shrinkage provides a convenient method for holding the tubular membrane in place and defining the diffusion area, but is not essential to the electrode. For example, a solid polymer capillary membrane can be stretched over the needle and held in place by the elastic properties of the polymer.

If desired, the membrane can be recessed into an annulus around the circumference of the needle. Such recessing is particularly appropriate where the membrane has a thick wall which might otherwise hinder insertion of the invasive probe electrode through the skin.

The invasive probe electrode of this invention can be employed with a reference electrode and optional counter electrode attached externally to the skin of the subject. The combination of the invasive probe electrode and an external electrode is surprisingly effective.

The external electrode can be located at any convenient point (where the potential drop is not significant) and is then moreover separated by the skin from possible electrochemically interfering substances in the tissues and also prevented from contributing potentially toxic components, such as silver, silver chloride, to such tissues.

In an alternative preferred construction, the reference electrode and optional counter electrode are constructed as part of the delivery system for an indirectly inserted invasive probe electrode. There is no difficulty in locating the further electrode so that it can come in to contact with the blood of the subject upon insertion of the cannula in to a blood vessel. Blood will tend to flow in the cannula, and for example a side port can be provided for the further electrode. Alternatively, the reference electrode and optional counter electrode can be incorporated with the probe electrode to form a unitary device.

The advantages of the present invention include the fact that a small invasive electrode can be placed within the tissues of the patient. Secondly, the electrode material seen by the body fluids can be located in various positions. For a glucose sensor, it is preferable to locate it just beneath the skin, being a position of minimum discomfort. However, as mentioned, it is possible to fabricate the invasive probe so that it is capable of placement into other tissues or organs, whereby a readout of a specific component level can be taken at a known in vivo location.

The invention will be further described by way of non-limiting example with reference to the accompanying drawings, in which:-

Figure 1 is a schematic diagram of a sensor system of this invention including two electrodes, not drawn to scale, but showing the respective electrode locations in relation to the skin of a subject;

Figure 2 shows diagrammically the stages (a) to (f) in the manufacture of the invasive needle probe portion of the system shown in Figure 1;

Figures 3a, 3b and 3c diagrammatically show optional features of membrane location for the invasive needle probe portion; and

Figures 4 and 5 show results obtained with the sensor system of Figure 1;

Figures 6a with 6b and 6c schematically illustrate stylised versions of alternative invasion probe electrodes of this invention with delivery system.

Figure 7 shows results (top half) obtained with a conventional assay in comparison with results (lower half) for a sensor made as shown in Figure 3c.

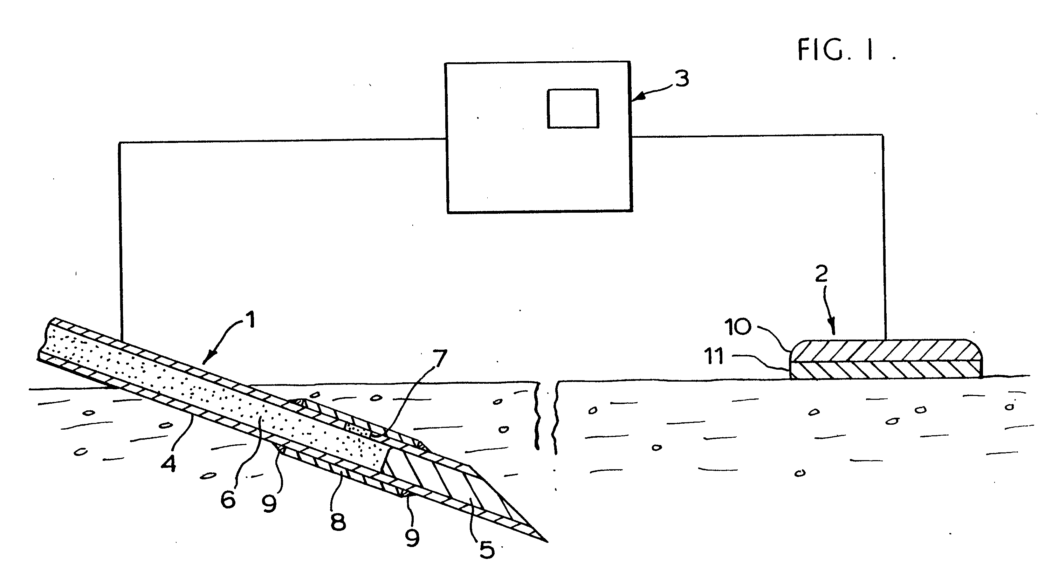

Figure 1 shows an invasive probe electrode 1 and a reference or counter electrode 2 for placement on the skin of the subject. These electrodes are connected to sensor processing circuitry 3 with read-out means, the circuitry being of known design for amperometric sensing.

The invasive probe 1 has been drawn to a larger scale than the other components since it has a more complex structure. It consists of a hypodermic needle 4 plugged at its outer end with a plug 5 of adhesive thermo-polymer such as a cyanoacrylate polymer, and containing within its hollow interior a mixture 6 of a particulate electrode material. By way of simple example, this electrode material can be a mixture of particulate carbon, dimethyl ferrocene, and glucose oxidase, such as forms the basis of a mediated enzyme electrode for detecting glucose. In the side wall of the needle there is formed an accurately dimensioned aperture 7, by a method described in more detail below.

A semi-permeable polymer membrane 8, preferably a phase inversion membrane, is shrink-fitted (for instance by solvent-shrinking) or simply pushed on and held in place by the elastic properties of the polymer, around the portion of the needle which includes the aperture 7. The adhesion to the needle is further assisted by end regions 9 of adhesive extending around the needle.

The needle and its conductive active electrode material contained therein is electrically connected to the processing means 3.

The conductive counter electrode/reference electrode 2 can be of generally conventional nature, and for example it can be an electrode of the kind adopted for electrocardiogram ("ECG") monitoring. Thus, the electrode 2 can consist of a metal disc 10, which in the present example is coated with a reference layer 11 of a mixture of silver and silver chloride particles.

In use of the sensor system of this invention, the electrode 2 is held in conventional manner to the surface of the skin so that the silver/silver chloride particles are in good contact. The probe electrode 1 is inserted beneath the skin so that the aperture 7, and thus the active electrode material, can come in to contact with body fluids, such as whole blood, in a known in vivo location.

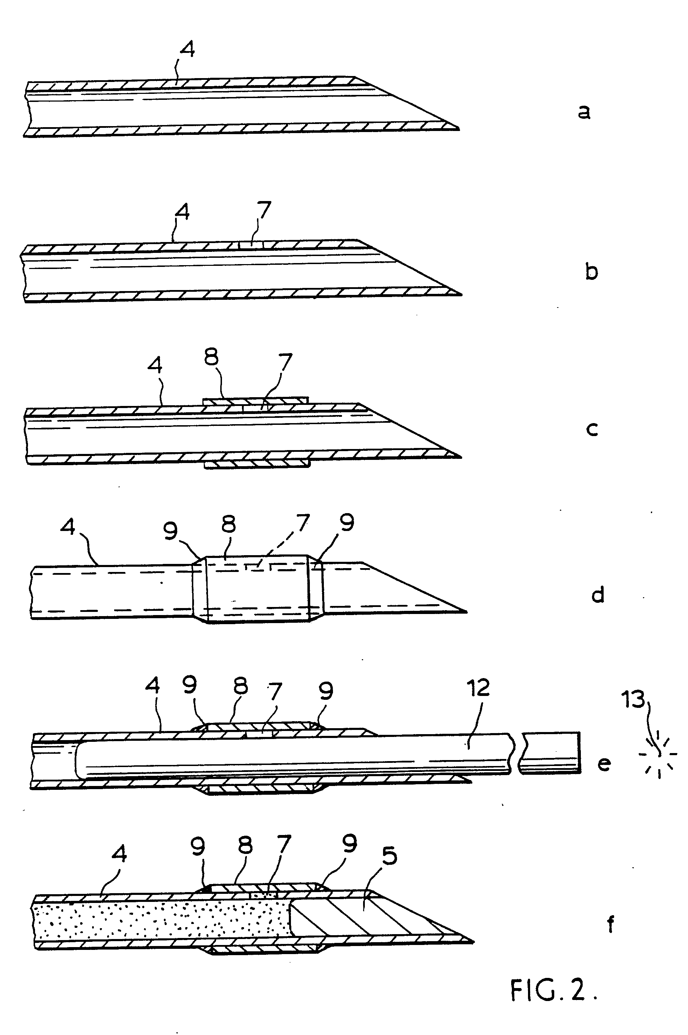

Figure 2 shows stages in the manufacture of the invasive needle probe electrode shown in Figure 1.

Figure 2a shows in section the outer end of a standard hypodermic needle 4.

Figure 2b shows the outer end of the needle 4 in section as before, with an aperture 7 drilled diametrically through one wall. The aperture 7 may be circular or somewhat elongate, but is of accurate reproducible dimension with essentially perpendicular side walls. A minimum of wall material is removed in order to preserve needle strength.

The adoption of diametrical drilling, with perpendicular side walls, is different from known drilling techniques hitherto adopted for drilling of syringe needles. Conventionally, needles with a perforated side wall have been made by grinding a recess over an array of parallel needles so that each needle becomes slotted. The slots are not accurately sized, but for the known uses this lack of exact dimensions is not important. Furthermore, the grinding technique can seriously weaken the needle.

For the purposes of the present invention, however, the environment must see an accurate area of active electrode material. To this end, the adoption of diametrical drilling is particularly preferred. Diametrical drilling is typically effected by sandblasting an accurately dimensioned aperture 7 through the needle wall 4. Suitable sandblasting equipment comprises, for example, the equipment available from Texas Airsonics Ltd of New Jersey, USA, under the Registered Trade Mark JETSONIC. Sandblasting of an aperture gives a stronger construction than the ground slotted needles known for other purposes.

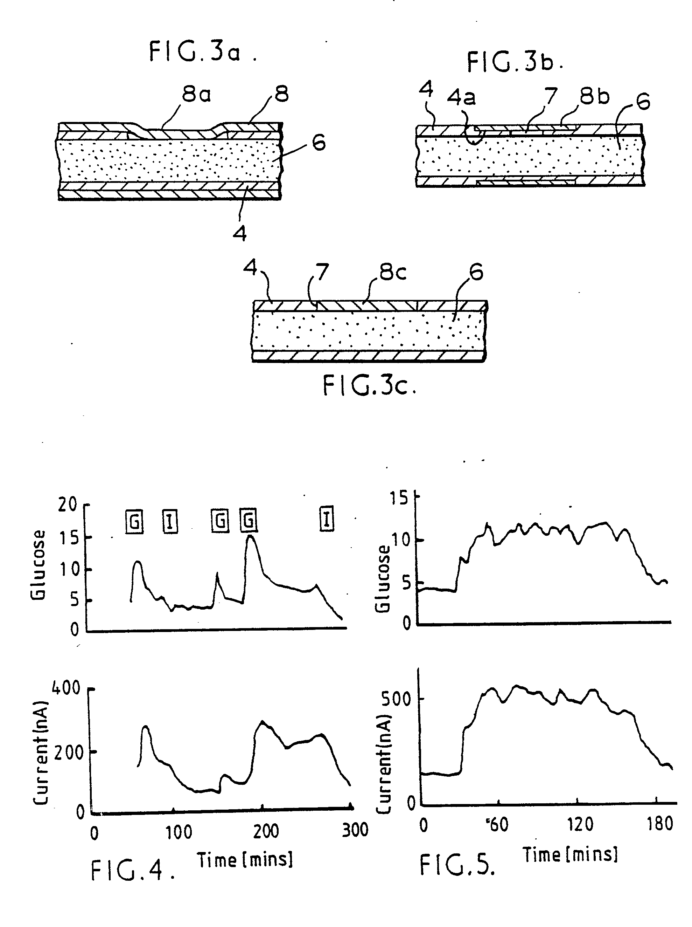

Figure 2c shows in section the side apertured needle 4 with a fitted sleeve 8 established around its exterior. The sleeve is of a thermopolymer permeable to solute molecules. Typically, the membrane depresses over the aperture at 8a, as shown in Figure 3a. The membrane can itself be located in a surface recess 4a as shown in Figure 3b.

In a further alternative, shown in Figure 3c, the membrane is cast in situ in the aperture. To this end, a stainless steel or other rod is inserted as a tight fit through one end of a needle with side aperture, and membrane solution is dropped in to the sidewall aperture. The needle with inserted rod is then immersed in a gelation bath to give the in situ membrane. The rod is removed, the needle filled with electrode material and sealed.

Figure 2d shows in elevation the additional securing of the sleeve 8 of polymer membrane by lines of adhesive 9 at each end of the sleeve. This securing is effected by turning the needle and allowing the adhesive to be applied from a small-diameter nozzle. There is thus achieved a tightly fitting and securely held membrane covering the accurately dimensioned electrode window. If desired, adhesive 9 can be applied to the needle as a circular band to either side of the aperture, before positioning the capillary-tube membrane, so as to anchor the membrane more securely. A thin layer of a hot melt adhesive can also be used.

Figure 2e shows in section an important feature of an accurate filling system. There is introduced into the opening of the needle a thin metal rod 12. The electrode materials are pumped in moist or slurry form (comprising for example, carbon, a ferrocene, and the requisite enzyme) into the hollow body of the needle. The metal rod 12 moves (to the right as shown in the drawings) and eventually occludes a photodiode 13 as shown. The system is so set up that at this point the inner end of the metal rod 12 defines the total extent of the electrode chamber.

Figure 2f shows the completed electrode. The metal rod 12 has been removed and a body of polymeric adhesive is incorporated in melt form to solidify and constitute a plug 5 at the end of the hollow needle 4.

The limiting of the extent of the electrode chamber using rod 12 and diode 13 is done mainly to allow a clean space for the plug 5. If alternatively the aperture end of the needle is filled with electrode material, and then this electrode material is pushed back by the liquid plug material, then the internal surface of the needle, at the aperture end, tends to be contaminated and the plug sometimes does not adhere so well.

The performance of the sensor system of Figure 1 was evaluated by in vivo studies on rats and on humans.

## Rats

Sprague-Dawley rats weighing 200-350g were studied. they were anaesthetised with 0.1ml sodium pentobarbitone (60mg/ml)100g weight which was given intra-abdominally. A jugular vein was catheterised and kept open with heparinised normal saline. Blood sampling was carried out at 5 minutes intervals from a tail vein, and the blood glucose was assessed.

In these studies, the plasma glucose concentrations were modulated up and down by using small quantities of 50% glucose or up to 1 unit of insulin intravenously through the jugular cannula, and further pentobarbitone was administered intravenously as necessary. The current was detected by two methods. The studies lasted up to 6 hours and were terminated with a lethal dose of pentobarbitone.

Figure 4 typifies the results when simultaneously the glucose level was measured and the electrode current recorded in rats. The modulation of the plasma glucose level was achieved with glucose "G" and with insulin "I", as indicated. It will be seen that there is a strong correlation of current with plasma glucose.

## Men

Volunteers were studied in a fasting state. A cannula was inserted in a distal vein kept arterialised by a warming device and the blood was continuously withdrawn at a rate of 0.5ml/min using a rotary pump. The blood was heparinised through a concentric cannula with the heparin pumped retrogradely by the same pump connected to a hydraulic reduction device. The subjects were studied for 30 minutes in the basal state and then the whole-blood glucose concentration was held at l0.9mmol/l whole blood (equivalent to 12mmol/l plasma concentration) by means of a bolus injection and the use of a variable infusion of 20% glucose controlled by an iterative computer programme. These studies lasted up to 4.5 hours.

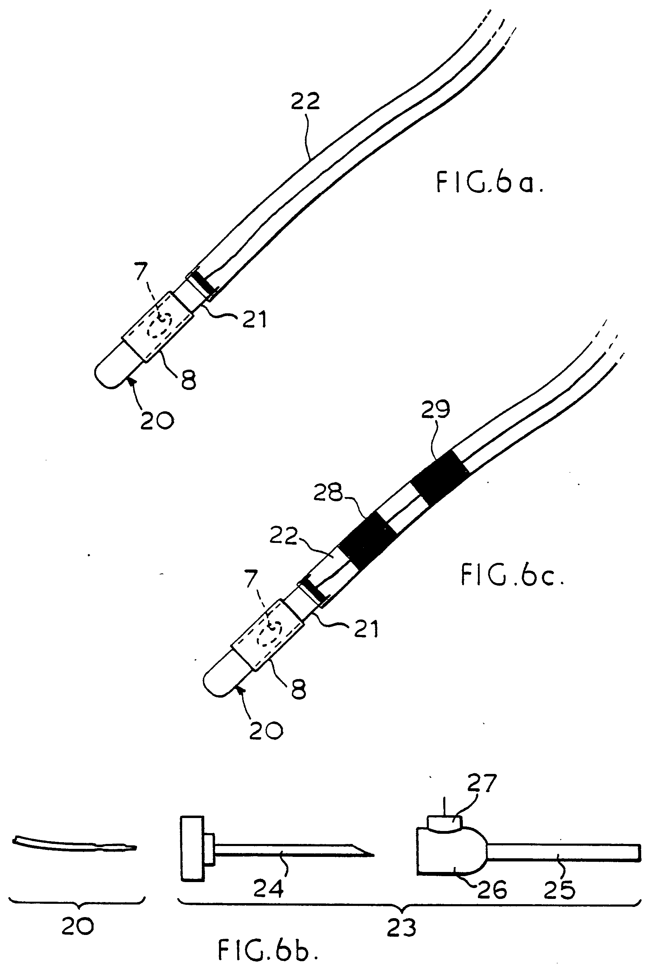

Figure 5 typifies the results when simultaneously the glucose level was measured and the electrode current recorded for subjects whose plasma glucose was clamped from basal to 10.9mmol/l whole blood concentration and then allowed to revert to basal again. A strong correlation was again found of current with glucose level.

The speed of response was high: one human subject had oscillations of glucose during the hyperglycaemic clamp and the current changes were very fast and showed good concordance with the measured glucose.

To examine whether the glucose sensor lagged behind the measured plasma response, cross-correlation analysis was carried out on the studies in man, where 2 minute sampling made phase-lag analysis possible. No phase lag was detectable; since peak correlation was at the point of no phase difference. It was also established that hysteresis was not a problem, hysteresis being a measure of the accuracy of the method on the upswing and down-swing .

There is no strong disadvantage in placing the electrode 2 close to the point of in vivo investigation, and indeed in one form of the invention the two electrodes can be mechanically associated so that the needle enters the subject in the region of the counter electrode, for instance by passing through a central hole in the counter electrode. Such an arrangement, for example, might be envisaged for an insulin-deficient patient who needed constant monitoring of the blood glucose level and constant controlled supply of insulin into subcutaneous tissue or fat. To this end, a feedback and supply device can be readily associated with the invasive probe electrode and external second electrode.

The system can also employ deeper or more internal probe electrodes, such as by catheter delivery systems or the like, as shown in Figure 6, and generally provides a way of introducing into a living subject a known area of active electrode material for specific interaction with an analyte to be investigated at that location.

Referring to Figures 6a and 6b, an invasive probe electrode 20 includes a short length of tube 21 with electrode fill and membrane 8 covering aperture 7, and a connecting lead 22 comprising a wire within plastic tubing. The electrode 14 is for use with a delivery system 23 based on an intravenous cannula. The delivery system 23 comprises a needle 24 and a cannula 25 which is a snug fit on the needle. The cannula 25 extends from a housing 26 which includes a side port 27 containing a reference electrode within a plug of hydrophilic polymer such as polyhydroxyethyl methacrylate, gelatin, or agarose.

In use, the needle 24 is housed within the cannula 25 and the needle tip extends beyond the cannula. The needle pierces the skin and places the cannula in to the vein. The needle 24 is withdrawn, and the invasive probe electrode is placed down the cannula, allowing readings to be taken.

The performance of the electrode 20 of Figure 6a was evaluated by in vivo studies on a human volunteer.

A cannula was inserted in a distal vein of a male volunteer. The vein was kept arterialised by a warming device and the blood was continuously withdrawn through a length of silicone rubber tubing at a rate of 0.5 ml/min using a Watson Marlow rotary pump. The blood was heparinised through a concentric cannula with the heparin pumped retrogradely by the same Watson Marlow pump connected to a hydraulic reduction device. Four glucose sensors were studied simultaneously during the experiment. They were situated in the silicone tubing between 3 -6 cm from the connection point of the tubing to the cannula. The reference and counter electrodes were positioned in the same tubing between 1-2 cm from the sites of the four sensors. The subject was studied for about 40 minutes in the basal state and then the whole-blood glucose concentration was clamped at 10 mmol/l by means of a bolus injection and the use of a variable infusion of 20% glucose, controlled by an iterative computer programme.

The change in blood glucose levels was measured by a reference assay and compared with changes in sensor response over the period of the experiment. Comparing actual glucose levels with the four sensor responses shows good correlation over the period of the experiment, with a rapid response time in all four cases. There also appears to be no obvious change in basal current over the experiment which probably indicates that very little membrane fouling is taking place.

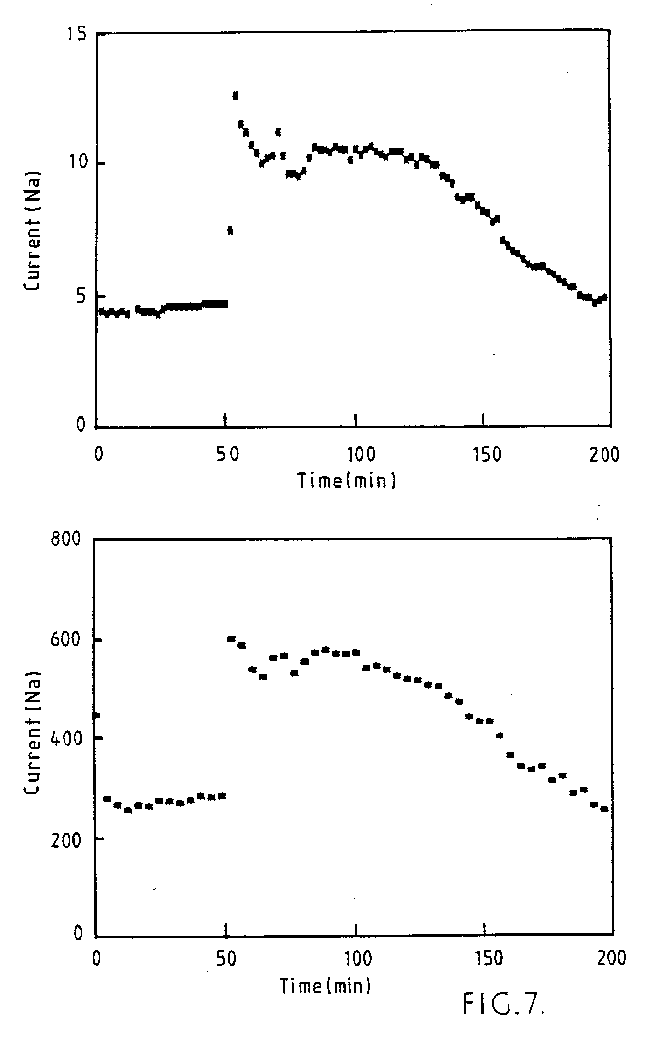

Figure 7 typifies results obtained using sensors of this invention as shown in Figure 3c inserted by a catheter system similar to that of Figure 6b, in comparison with results for a conventional assay system. The change in glucose level with time is accurately followed by the devices of the present invention.

Figure 6c illustrates a variation of the system of Figures 6a and 6b, with the reference electrode 28 and counter electrode 29 incorporated with the probe electrode 20 to form a unitary device.

While the specific examples given above by reference to the drawings relate to mediator-containing electrodes, the invention may also be embodied in the form of a non-mediated electrode or a direct amperometric electrode.

## Claims

1. An amperometric invasive probe electrode for detecting an analyte, which electrode comprises a fill of electrode material contained in a generally cylindrical closed tube having an aperture protected by a membrane cover.

2. An invasive probe electrode according to claim 1, which is a needle capable of piercing skin.

3. An invasive probe electrode according to claim 1, together with a delivery system for indirect introduction of the electrode.

4. An invasive probe electrode according to claim 1 which is a single-use disposable electrode.

5. An invasive probe electrode according to claim 1 wherein the electrode material comprises a mediated enzyme system for amperometric detection of an analyte.

6. An invasive probe electrode according to claim 1, wherein the membrane cover comprises a semi-permeable, microporous membrane which is a phase inversion membrane.

7. An invasive probe electrode according to claim 1, wherein the membrane is cast in situ at the aperture.

8. An invasive probe electrode according to claim 1, wherein the membrane is cast separately as a tube and placed around the aperture.

9. A sensor system which comprises:

   (a) the amperometric invasive probe electrode of claim 1;

   (b) at least one further electrode comprising a reference electrode and optionally a counter electrode;
and

   (c) electrical circuitry for connection between the electrodes to amperometrically detect the analyte.

10. A sensor system according to claim 9, wherein the reference electrode and optional counter electrode are external electrodes.

FIG. I.

EP 0 320 109 A1

FIG. 2.

FIG.3a.

FIG.3b.

FIG.3c.

FIG.4.    Time [mins]

FIG.5.    Time [mins]

22

FIG.6a.

7

21

8

20

29

28

22

7

FIG.6c.

21

8

20

27

24

26   25

20

23

FIG.6b.

FIG.7.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 206 531 (THE REGENTS OF THE UNIVERSITY OF CALIFORNIA) * Abstract; page 4, line 13 - page 6, line 3; page 7, lines 14-30; page 8, lines 8-26; figures 1-9 * | 1,2,5,6 ,9 | A 61 B 5/00 |
| Y | | 3,4,7,8 ,10 | |
| D,Y | EP-A-0 127 958 (GENETICS INTERNATIONAL) * Abstract; page 16, lines 1-6; page 23, line 16 - page 24, line 11; figures 1-4 * | 4,7,8 | |
| Y | US-A-3 415 730 (I.A. HADDAD) * Column 2, lines 46-66; figure 1 * | 3 | |
| Y | US-A-4 499 901 (J.J. CHANG et al.) * Abstract; column 2, line 57 - column 3, line 15; figures 1,2 * | 10 | |
| A | EP-A-0 120 108 (HONEYWELL AND PHILIPS MEDICAL ELECTRONICS BV) * Abstract; page 2, line 15 - page 4, line 16; figures 3,4 * | 1,9 | TECHNICAL FIELDS SEARCHED (Int. Cl.4) A 61 B C 12 M G 01 N |

The present search report has been drawn up for all claims

| Place of search | Date of completion of the search | Examiner |
|---|---|---|
| THE HAGUE | 08-03-1989 | HUNT,B.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    & : member of the same patent family, corresponding document

EPO FORM 1503 03.82 (P0401)